# EUROPEAN PATENT APPLICATION

(11) **EP 3 954 319 A1**
(43) Date of publication of application: **16.02.2022**
(21) Application number: 20787495.9
(22) Date of filing: 01.04.2020
(51) Int. Cl.: A61B 34/35

(54) **SURGICAL OPERATION SYSTEM AND METHOD FOR CONTROLLING SURGICAL OPERATION SYSTEM**

(30) Priority: 11.04.2019 JP 2019075278
(71) Applicant: Kawasaki Jukogyo Kabushiki Kaisha, Hyogo 650-8670 (JP)
(72) Inventor: TANAKA, Hideki, Hyogo 650-8670 (JP)
(74) Representative: Dehns
(86) International application number: PCT/JP2020/015078
(87) International publication number: WO 2020/209165

(57) **Abstract**

A surgical operation system (100) includes an operation unit (1), a hand control unit (42), and a controller (3). In a normal operation mode, a normal operation command generator (55) calculates a target posture of a surgical instrument (23) based on a displacement of a handle (65), the displacement being detected by a detector (75), and generates a normal operation command to operate a wrist joint (33) such that the surgical instrument (23) takes the target posture. In a bendable joint operation mode, a bendable joint operation command generator (56) generates a bendable joint operation command to change a bending angle of a bendable joint (32) based on the displacement of the handle (65), the displacement being detected by the detector (75).

## Description

### Technical Field

The present invention relates to a surgical operation system and a method of controlling a surgical operation system.

### Background Art

Conventionally, there has been a known apparatus for use in a minimally invasive surgery (see Patent Literature 1, for example).

The apparatus includes an arm to which a surgical tool is fixed. The surgical instrument is controlled through operations of a handle by a surgeon.

### Citation List

### Patent Literature

PTL 1: Japanese Laid-Open Patent Application Publication No. 2007-125404

### Summary of Invention

### Technical Problem

However, the apparatus of Patent Literature 1 has a problem in that the distal end portion of the arm, which is positioned inside the body of a patient, has limited degrees of freedom, and therefore, it is in some cases difficult for the distal end portion of the arm to access a surgical site.

### Solution to Problem

In order to solve the above-described problems, a surgical operation system according to one aspect of the present invention includes: an instrument manipulator including a shaft, a bendable joint, a wrist joint, and a surgical instrument that are connected and arranged in this order from a proximal end portion toward a distal end portion of the instrument manipulator, the instrument manipulator further including a wrist joint driver that drives the wrist joint based on a normal operation command including a command that specifies a target position of the wrist joint, and a bendable joint driver that drives the bendable joint based on a bendable joint operation command that specifies a target bending angle of the bendable joint; a master console including a base, a hand control unit including a handle that is displaceable relative to the base and a detector that detects a displacement of the handle, and an operation mode selection inputter that receives an operation of inputting a selection command to select one operation mode from operation modes including a normal operation mode and a bendable joint operation mode; and a controller including an operation mode setter that sets the operation mode to the one operation mode corresponding to the selection command, a normal operation command generator that generates the normal operation command, and a bendable joint operation command generator that generates the bendable joint operation command. In the normal operation mode, the normal operation command generator calculates a target posture of the surgical instrument based on the displacement of the handle, the displacement being detected by the detector, and generates the normal operation command to operate the wrist joint such that the surgical instrument takes the target posture. In the bendable joint operation mode, the bendable joint operation command generator generates the bendable joint operation command to change the bending angle of the bendable joint based on the displacement of the handle, the displacement being detected by the detector.

According to this configuration, since the instrument manipulator includes the bendable joint, the motion range of the instrument manipulator can be widened, and the degree of freedom of posture of the instrument manipulator can be increased. Since the bendable joint can be operated by using the handle, an increase in the operational burden on a surgeon due to the addition of the operation of the bendable joint can be suppressed. This makes it possible to reduce a surgery time.

### Advantageous Effects of Invention

The present invention has an advantage of making it possible to reduce a surgery time.

### Brief Description of Drawings

FIG. 1 schematically shows an example of an overall configuration of a surgical operation system according to Embodiment 1.
FIG. 2 is a perspective view showing a configuration example of an instrument manipulator of the surgical operation system of FIG. 1.
FIG. 3 is an essential-part enlarged perspective view showing a configuration example of a distal-side portion of a hand control unit of the surgical operation system of FIG. 1.
FIG. 4 is a block diagram schematically showing a configuration example of a control system of the surgical operation system of FIG. 1.
FIG. 5 is a perspective view showing an example of an operation of a bendable joint of the surgical operation system of FIG. 1.
FIG. 6 shows an example of an operation of the bendable joint of the surgical operation system of FIG. 1.
FIG. 7 is a perspective view showing a configuration example of an instrument manipulator of a surgical operation system according to Embodiment 2.

### Description of Embodiments

A surgical operation system according to one aspect includes: an instrument manipulator including a shaft, a bendable joint, a wrist joint, and a surgical instrument that are connected and arranged in this order from a proximal end portion toward a distal end portion of the instrument manipulator, the instrument manipulator further including a wrist joint driver that drives the wrist joint based on a normal operation command including a command that specifies a target position of the wrist joint, and a bendable joint driver that drives the bendable joint based on a bendable joint operation command that specifies a target bending angle of the bendable joint; a master console including a base, a hand control unit including a handle that is displaceable relative to the base and a detector that detects a displacement of the handle, and an operation mode selection inputter that receives an operation of inputting a selection command to select one operation mode from operation modes including a normal operation mode and a bendable joint operation mode; and a controller including an operation mode setter that sets the operation mode to the one operation mode corresponding to the selection command, a normal operation command generator that generates the normal operation command, and a bendable joint operation command generator that generates the bendable joint operation command. In the normal operation mode, the normal operation command generator calculates a target posture of the surgical instrument based on the displacement of the handle, the displacement being detected by the detector, and generates the normal operation command to operate the wrist joint such that the surgical instrument takes the target posture. In the bendable joint operation mode, the bendable joint operation command generator generates the bendable joint operation command to change the bending angle of the bendable joint based on the displacement of the handle, the displacement being detected by the detector.

According to this configuration, since the instrument manipulator includes the bendable joint, the motion range of the instrument manipulator can be widened, and the degree of freedom of posture of the instrument manipulator can be increased. Since the bendable joint can be operated by using the handle, an increase in the operational burden on a surgeon due to the addition of the operation of the bendable joint can be suppressed. This makes it possible to reduce a surgery time.

The wrist joint may be a joint with two degrees of freedom or three degrees of freedom.

This configuration makes it possible to widen the motion range of the surgical instrument.

The instrument manipulator may further include: a first arm on a proximal end portion side of the instrument manipulator; and a first arm driver that drives the first arm based on the normal operation command, which further includes a command that specifies a motion of the first arm. In the normal operation mode, the normal operation command generator may generate the normal operation command to operate the first arm and the wrist joint such that the surgical instrument takes the target posture at the target position.

This configuration makes it possible to widen the motion range of the surgical instrument.

The normal operation command generator may generate the normal operation command to control a position and a posture of the surgical instrument while keeping a center axis of the shaft passing through a preset remote center.

According to this configuration, the instrument manipulator can be swung about the remote center, and thereby a burden on a patient can be reduced.

Hereinafter, embodiments of the present invention are described with reference to the drawings. It should be noted that the present invention is not limited by these embodiments. In the drawings, the same or corresponding elements are denoted by the same reference signs, and repeating the same descriptions is avoided below.

### (Embodiment 1)

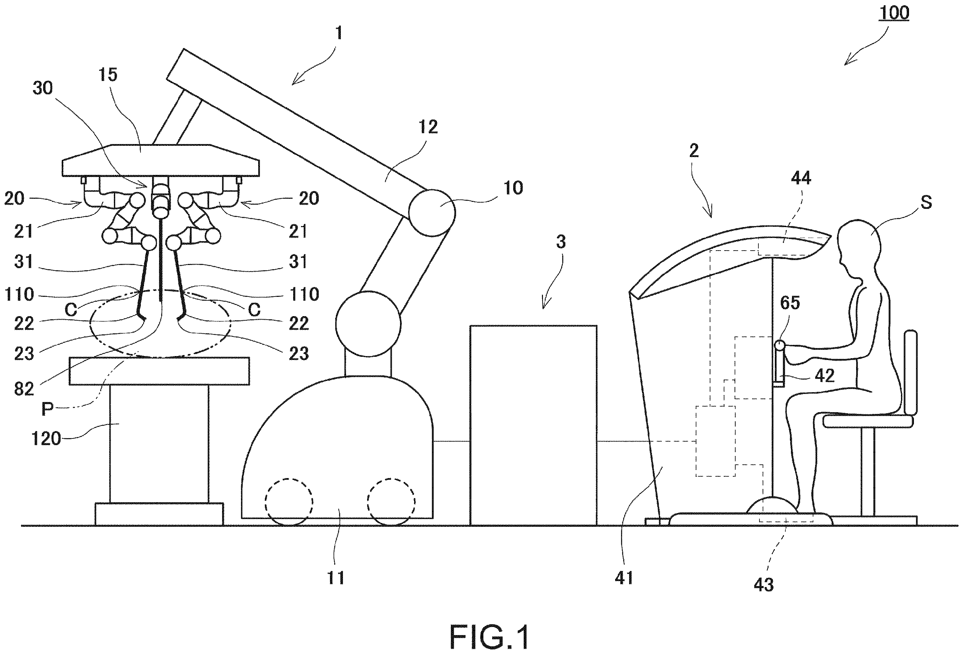

FIG. 1 schematically shows an example of an overall configuration of a surgical operation system 100 according to Embodiment 1.

The surgical operation system 100 shown in FIG. 1 is intended for performing a laparoscopic surgery. The surgical operation system 100 includes an operation unit 1, a master console 2, and a controller 3. Hereinafter, components of the surgical operation system 100 are described in detail.

### [Operation Unit 1]

The operation unit 1 serves as an interface between the surgical operation system 100 and a patient P. In an operating room, which is a sterile field, the operation unit 1 is disposed by the side of a surgical table 120, on which the patient P lies.

The operation unit 1 includes instrument manipulators 20, an endoscope manipulator 30, and a positioner 10. The positioner 10 positions the instrument manipulators 20 and the endoscope manipulator 30 in relation to the patient P.

The positioner 10 has a function as a supporting device that supports and guides the instrument manipulators 20 and the endoscope manipulator 30. The positioner 10 includes: a truck 11; a supporting arm 12 supported by the truck 11; and an arm base 15 provided on the distal end portion of the supporting arm 12. The configuration of the positioner 10 is not limited to the present embodiment. The positioner 10 may be configured differently, so long as the positioner 10 can position the instrument manipulators 20 and the endoscope manipulator 30 at their respective target positions with high precision. The supporting arm 12 is a vertical articulated robotic arm. The supporting arm 12 includes: servomotors (not shown) provided on respective joints; and a driver (not shown) including a power transmission mechanism that transmits motive power of the servomotors to the respective joints.

### (Instrument Manipulators 20)

Each instrument manipulator 20 includes a first arm 21, a shaft 31, a bendable joint 32, a wrist joint 33, and a surgical instrument 23. The first arm 21, the shaft 31, the bendable joint 32, the wrist joint 33, and the surgical instrument (jaws) 23 are connected and arranged in this order from the proximal end portion toward the distal end portion of the instrument manipulator 20.

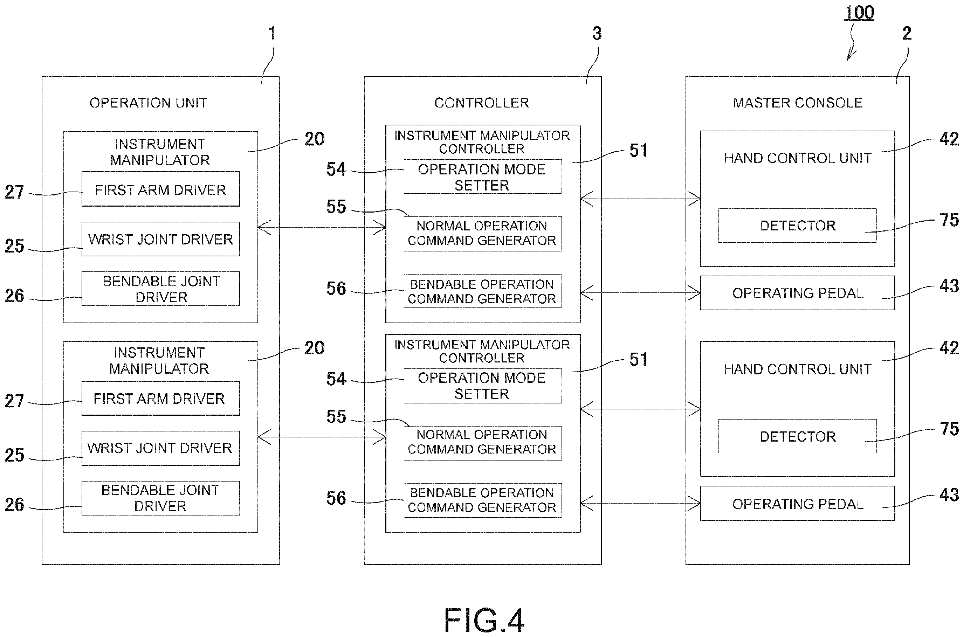

For example, the first arm 21 is a vertical articulated robotic arm. The first arm 21 includes: servomotors provided on respective joints; a first arm driver 27 (see FIG. 4) including a power transmission mechanism that transmits motive power of the servomotors to the respective joints. The first arm 21 is, for example, a six-axis or seven-axis robotic arm.

The shaft 31 is a stiff tube, and the center axis of the shaft 31 extends linearly. The middle portion of the shaft 31 is inserted in a trocar 110. The shaft 31 couples the first arm 21 positioned outside the patient's body and, for example, the bendable joint 32 positioned in a body cavity of the patient P.

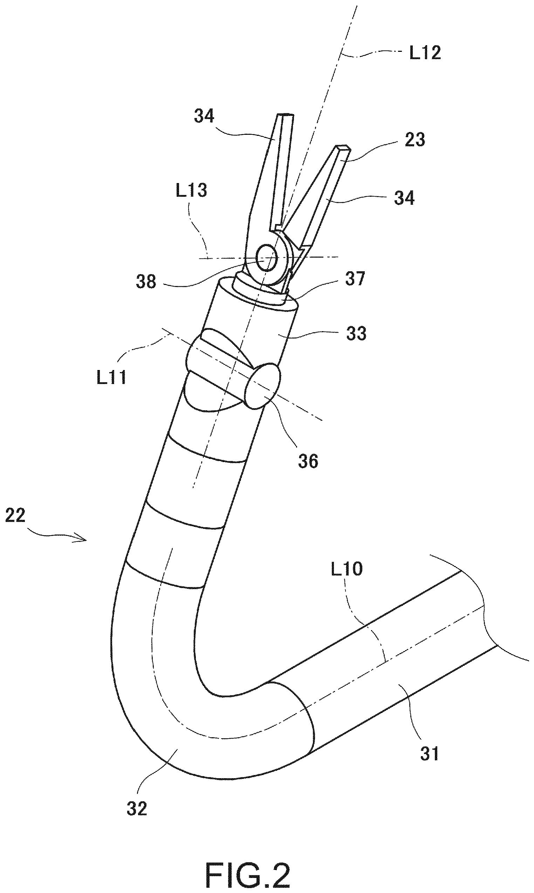

FIG. 2 is a perspective view showing a configuration example of a second arm 22. The second arm 22 includes the bendable joint 32 and the wrist joint 33. The bendable joint 32 and the wrist joint 33 are connected and arranged in this order from the proximal end portion toward the distal end portion of the second arm 22. It is not essential that the bendable joint 32 and the wrist joint 33 be directly coupled to each other. Another component may be interposed between the bendable joint 32 and the wrist joint 33.

The bendable joint 32 is a joint that makes a bending motion in a direction orthogonal to the longitudinal direction of a part of the instrument manipulator 20, the part ranging from the shaft 31 to the distal end portion of the instrument manipulator 20 (i.e., makes a bending motion in a direction orthogonal to the direction in which a center axis L10 extends). The bendable joint 32 is bendable in a direction orthogonal to the longitudinal direction of the shaft 31 (i.e., bendable in a radial direction with respect to the center axis of the shaft 31). The definition of the term "bend" herein also encompasses the meanings of "flex", "curve", "fold", etc. The bendable joint 32 has a function as a redundant axis of the instrument manipulator 20, and allows the instrument manipulator 20 to have a redundant degree of freedom.

The wrist joint 33 is a joint that makes a bending motion at least in a direction orthogonal to the longitudinal direction of the aforementioned part ranging from the shaft 31 to the distal end portion of the instrument manipulator 20 (i.e., in a direction orthogonal to the direction in which the center axis L10 extends). The wrist joint 33 is bendable in a direction orthogonal to the longitudinal direction of the shaft 31. In the present embodiment, the wrist joint 33 is a joint with three degrees of freedom, and includes three rotational joints (a first joint 36, a second joint 37, and a third joint 38). The first joint 36 is bendable in a first bending direction. The first bending direction is orthogonal to the direction in which the distal end portion of the bendable joint 32 extends. The second joint 37 is a pivot, and is rotatable about an axis L12. The third joint 38 is bendable in a second bending direction. The second bending direction is orthogonal to the first bending direction and the direction in which the distal end portion of the bendable joint 32 extends. The first joint 36, the second joint 37, and the third joint 38 are connected and arranged in this order in a direction from the proximal end portion toward the distal end portion of the instrument manipulator 20. However, the order in which these joints 36 to 38 are arranged is not limited to this example.

The surgical instrument 23 is an instrument that is connected to the wrist joint 33. The surgical instrument 23 is, for example, forceps including a pair of jaws 34. The surgical instrument 23 is not limited to forceps, but may be a surgical tool such as a gripper, scissors, a stapler, a needle holder, or an electric surgical knife. Alternatively, the surgical instrument 23 may be an electrically driven device such as an electrosurgical electrode, a transducer, or a sensor. Further alternatively, the surgical instrument 23 may be a fluid feeding nozzle for use in, for example, suction, gas insufflation, irrigation, feeding of a treatment fluid, accessory introduction, or biopsy extraction.

In addition to the above-described first arm driver 27, the instrument manipulator 20 includes a wrist joint driver 25 (see FIG. 4) and a bendable joint driver 26 (see FIG. 4). The wrist joint driver 25 drives the wrist joint 33. The bendable joint driver 26 drives the bendable joint 32. A normal operation command includes a command that specifies a target position of the wrist joint 33. The wrist joint driver 25 drives the wrist joint 33 based on the normal operation command. The bendable joint driver 26 drives the bendable joint 32 based on a bendable joint operation command that specifies a bending angle of the bendable joint 32. The wrist joint driver 25 and the bendable joint driver 26 are operated by pulling force and/or tensile force of an unshown electrically-operated wire. The term "bending angle" of a joint herein may mean an angle formed by the center axis of a portion that is located toward the proximal end side from the joint and the center axis of a portion that is located toward the distal end side from the joint. The normal operation command further includes a command that specifies a motion of the first arm 21. The first arm driver 27 drives the first arm 21 based on the normal operation command.

### (Endoscope Manipulator 30)

As shown in FIG. 1, the distal end portion of the endoscope manipulator 30 is provided with an endoscope camera unit 82. The configuration of the endoscope manipulator 30 is substantially the same as the configuration of the instrument manipulator 20, except the surgical instrument 23 provided on the distal end portion of the instrument manipulator 20. That is, the configuration of the endoscope manipulator 30 can be described by replacing, in the above description of the instrument manipulator 20, the surgical instrument 23 provided on the distal end portion of the instrument manipulator 20 with the endoscope camera unit 82.

### [Master Console 2]

The master console 2 serves as an interface between the surgical operation system 100 and a surgeon S. The master console 2 is an apparatus for operating the operation unit 1. In the operating room, the master console 2 is set by the side of, or away from, the surgical table 120. Alternatively, the master console 2 is set outside the operating room.

The master console 2 includes: a base 41; a pair of left and right hand control units 42 mounted to the base 41; operating pedals 43 mounted to the base 41; and a display module 44.

Each hand control unit 42 includes a first control unit and a second control unit 60. The first control unit is supported by the base 41. The second control unit 60 is connected to the first control unit. The unshown first control unit includes links that are coupled to each other via prismatic joints or rotary joints, and the first control unit has three degrees of freedom of position. The second control unit 60 includes links that are coupled to each other via prismatic joints or rotary joints, and the second control unit 60 has three degrees of freedom of posture. The second control unit 60 includes a handle 65, which the surgeon S holds at the time of inputting an operation command. Accordingly, the handle 65 is displaceable relative to the base 41. Thus, overall, the hand control unit 42 has, at least, three degrees of freedom of position and three degrees of freedom of posture, i.e., six degrees of freedom. The hand control unit 42 is configured such that the handle 65 can be moved with six degrees of freedom relative to the base 41. The hand control unit 42 includes a detector 75, which detects three degrees of freedom of position of the first control unit, three degrees of freedom of posture of the second control unit 60, and one degree of freedom of holding of the handle 65, which will be described below. The detector 75 is a sensor that detects a displacement of each arm joint and a distance between a pair of annular bodies 66a of the handle 65. The pair of annular bodies 66a will be described below. The detector 75 is, for example, a potentiometer or a rotary encoder.

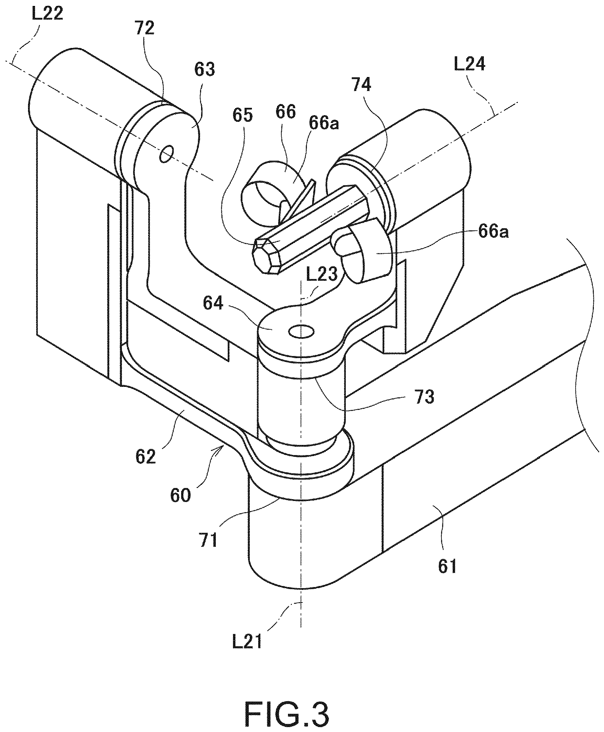

FIG. 3 is an essential-part enlarged perspective view showing a configuration example of the second control unit 60 of the hand control unit 42.

The second control unit 60 of the hand control unit 42 includes: four links (a first link 61, a second link 62, a third link 63, and a fourth link 64), which are connected in a line; the handle 65; and four joints (a first joint 71, a second joint 72, a third joint 73, and a fourth joint 74), each of which couples adjacent links to each other in a rotatable manner, or couples a link and the handle 65 to each other in a rotatable manner. The handle 65 is connected to the fourth link 64 via the fourth joint 74. The first joint 71 couples the second link 62 to the first link 61, such that the second link 62 is rotatable about a control unit first axis L21. The second joint 72 couples the third link 63 to the second link 62, such that the third link 63 is rotatable about a control unit second axis L22. The third joint 73 couples the fourth link 64 to the third link 63, such that the fourth link 64 is rotatable about a control unit third axis L23. The fourth joint 74 couples the handle 65 to the fourth link 64, such that the handle 65 is rotatable about a control unit fourth axis L24. The control unit first axis L21 and the control unit third axis L23 extend parallel to each other. The control unit second axis L22 is orthogonal to the control unit first axis L21. The control unit fourth axis L24 crosses a plane in which the control unit first axis L21 and the control unit second axis L22 are positioned. Accordingly, the handle 65 can be placed in any intended posture. The posture of the handle 65 is detected by the detector 75 by using an angular position of each of the four joints. A displacement of each joint, the displacement being detected by the detector 75, is outputted to the controller 3.

The handle 65 includes an instrument motion inputter 66. The instrument motion inputter 66 includes the pair of annular bodies 66a, which allow the thumb and the middle finger of the surgeon S to be inserted therethrough, respectively. The instrument motion inputter 66 allows the handle 65 to have one degree of freedom of holding. A distance between the pair of annular bodies 66a, the distance being detected by the detector 75, is outputted to the controller 3. The controller 3 controls the third joint 38 to open/close the pair of jaws 34.

As shown in FIG. 1, each operating pedal (an operation mode selection inputter) 43 is disposed such that the operating pedal 43 is positioned near a foot of the surgeon S holding the handle 65. The operating pedal 43 is a pedal switch that receives an operation of inputting a selection command to select one operation mode from operation modes including a normal operation mode and a bendable joint operation mode. The operating pedal 43 includes a pedal body (not shown) and a sensor. The pedal body is swingable between a first angular position and a second angular position. The second angular position is reached when the pedal body is stepped on further from the first angular position. The pedal body is urged from the second angular position toward the first angular position. The sensor detects an angular position of the pedal body.

The display module 44 displays an image captured by the endoscope camera unit 82 of the endoscope manipulator 30.

### [Controller 3]

FIG. 4 is a block diagram schematically showing a configuration example of a control system of the surgical operation system 100.

As shown in FIG. 4, the controller 3 is communicably connected to the instrument manipulators 20 of the operation unit 1. The controller 3 is also communicably connected to the hand control units 42 and the operating pedals 43 of the master console 2. Further, the controller 3 is communicably connected to the endoscope manipulator 30 and the display module 44 (not shown). The controller 3 operates the instrument manipulators 20 and the endoscope manipulator 30 in response to operation commands received via the master console 2.

The controller 3 includes an arithmetic processor (not shown) such as a CPU and memories (not shown) such as a ROM and RAM The memories store therein, for example, programs executed by the arithmetic processor and various data. The arithmetic processor transmits and receives data to and from other apparatuses, such as the operation unit 1 and the master console 2. The arithmetic processor receives detection signals inputted from various sensors, and outputs control signals to respective control targets. The controller 3 performs processes for realizing the below-described functions as the controller 3 by reading out and executing software, such as the programs stored in the memories, by the arithmetic processor. The controller 3 may execute the processes by centralized control that is performed by a single computer, or may execute the processes by distributed control that is performed by multiple computers working in cooperation with each other. The controller 3 may be configured as, for example, a microcontroller or a programmable logic controller (PLC). The controller 3 includes two instrument manipulator controllers 51 corresponding to the pair of hand control units 42, respectively. The instrument manipulator controllers 51 are function blocks that are realized as a result of the arithmetic processor executing predetermined control programs stored in the memories. The controller 3 further includes an endoscope controller and a display module controller (not shown). The endoscope controller controls the endoscope manipulator 30. The display module controller controls the display module 44.

Each instrument manipulator controller 51 included in the controller 3 includes an operation mode setter 54, a normal operation command generator 55, and a bendable joint operation command generator 56.

The operation mode setter 54 sets the operation mode to one operation mode corresponding to the selection command inputted via the operating pedal 43. Specifically, when the surgeon S removes his/her foot from the pedal body and the sensor detects that the pedal body is positioned at the first angular position, the operation mode setter 54 sets the operation mode to the normal operation mode. The normal operation mode is an operation mode in which an operation of moving the surgical instrument 23 is performed. When the surgeon S steps on the pedal body and the built-in sensor detects that the pedal body is positioned at the second angular position, the operation mode setter 54 sets the operation mode to the bendable joint operation mode. The bendable joint operation mode is an operation mode in which an operation of bending the bendable joint 32 is performed.

In the normal operation mode, the normal operation command generator 55 calculates a target position and a target posture of the surgical instrument 23 based on a displacement of the handle 65, the displacement being detected by the detector 75. Then, the normal operation command generator 55 generates the normal operation command to operate the wrist joint 33 and the first arm 21 such that the surgical instrument 23 takes the target posture at the calculated target position. That is, the normal operation command specifies the target position of the wrist joint 33 and a motion of the first arm 21. The normal operation command generator 55 generates the normal operation command to control the position and the posture of the surgical instrument 23 while keeping the center axis of the shaft 31 passing through a preset remote center C. The target position and the target posture of the surgical instrument 23 may be represented by a homogeneous transformation matrix in a three-dimensional orthogonal reference coordinate system on the operation unit 1 side.

In the bendable joint operation mode, the bendable joint operation command generator 56 generates the bendable joint operation command to change the bending angle of the bendable joint 32 based on a displacement of the handle 65, the displacement being detected by the detector 75.

### [Example of Operations]

Next, an example of operations of the surgical operation system 100 is described.

First, in a state where the foot of the surgeon S is away from the operating pedal 43, the operation mode setter 54 sets the operation mode to the normal operation mode.

Next, when the surgeon S holds and moves the handle 65 to cause the surgical instrument 23 to access a surgical site, the normal operation command generator 55 performs a forward transformation process using joint positions detected by the detector 75 to calculate a homogeneous transformation matrix representing the position and the posture of the handle 65 in a three-dimensional orthogonal reference coordinate system on the master console 2 side. Then, the normal operation command generator 55 transforms the homogeneous transformation matrix representing the position and the posture of the handle 65 in the three-dimensional orthogonal reference coordinate system on the master console 2 side into a homogeneous transformation matrix representing a target position and a target posture of the surgical instrument 23 in the three-dimensional orthogonal reference coordinate system on the operation unit 1 side. Thereafter, the normal operation command generator 55 inversely transforms the target position and the target posture of the surgical instrument 23 into the joint positions of the first arm 21 and the joint positions of the wrist joint 33. Subsequently, the normal operation command generator 55 generates position commands that indicate the calculated joint positions of the first arm 21 as target positions, and further generates position commands that indicate the calculated joint positions of the wrist joint 33 as target positions. These position commands are included in the normal operation command. The controller 3 outputs the generated normal operation command to the first arm driver 27. In response, the first arm driver 27 positions each joint at its target position to position the surgical instrument 23 near the surgical site. Further, the controller 3 outputs the generated normal operation command to the wrist joint driver 25. In response, the wrist joint driver 25 positions each joint at its target position to change the posture of the surgical instrument 23 such that the surgical instrument 23 is directed toward the surgical site, and causes the surgical instrument 23 to access the surgical site. In this manner, the surgical instrument 23 can be brought into a position and a posture corresponding to the position and the posture of the handle 65 so that a surgery can be performed.

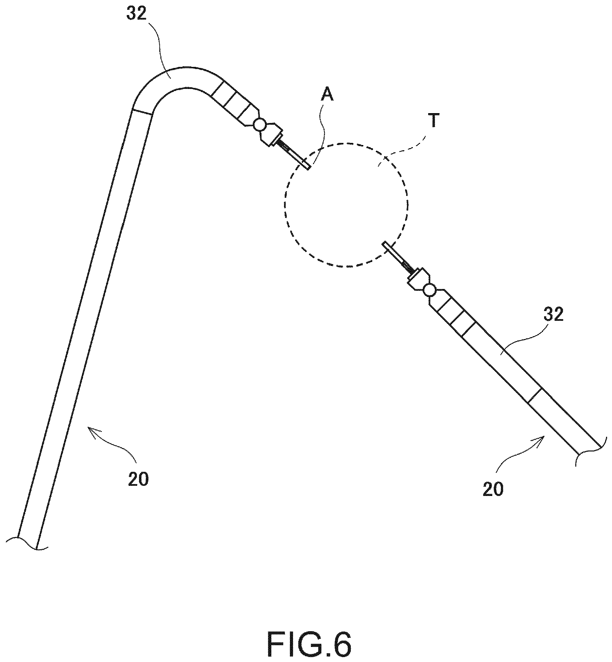

For example, as shown in FIG. 6, there may be a case where a portion A that is positioned at the back of the surgical site T as seen from the instrument manipulator 20 is to be accessed. In such a case, the portion A of the surgical site T cannot be properly accessed due to obstruction by other portions of the surgical site T. In this case, the bendable joint 32 is bent to change the posture of the entire wrist joint 33. Hereinafter, one example of an operation of bending the bendable joint 32 is described in detail.

When bending the bendable joint 32, first, the surgeon S steps on the operating pedal 43. As a result, the detector of the operating pedal 43 detects the state of the pedal, and the operation mode setter 54 sets the operation mode to the bendable joint operation mode.

Next, when the surgeon S holds and moves the handle 65 to change the posture of the handle 65 in a direction to bend the bendable joint 32, the bendable joint operation command generator 56 performs a forward transformation process using joint positions detected by the detector 75 to calculate a homogeneous transformation matrix representing the position and the posture of the handle 65 in the three-dimensional orthogonal reference coordinate system on the master console 2 side. Then, the bendable joint operation command generator 56 transforms the rotation matrix component of the homogeneous transformation matrix representing the posture of the handle 65 in the three-dimensional orthogonal reference coordinate system on the master console 2 side into a rotation matrix representing a target posture of the surgical instrument 23 in the three-dimensional orthogonal reference coordinate system on the operation unit 1 side. Then, the bendable joint operation command generator 56 calculates the bending angle of the bendable joint 32 based on the rotation matrix. In the three-dimensional orthogonal coordinate system on the operation unit 1 side, the z-axis is defined so as to extend in the forward moving direction of the surgical instrument 23, and a vector in the forward moving direction may be referred to as an approach vector. Also, the x-axis is defined relative to the z-axis such that, in the three-dimensional orthogonal coordinate system on the operation unit side, the bendable joint 32 is bent about an axis extending in the X-axis direction. Further, the y-axis is defined relative to the z-axis and the x-axis so as to form a right-handed system. Hereinafter, a method of calculating the bending angle of the bendable joint 32 is described in detail.

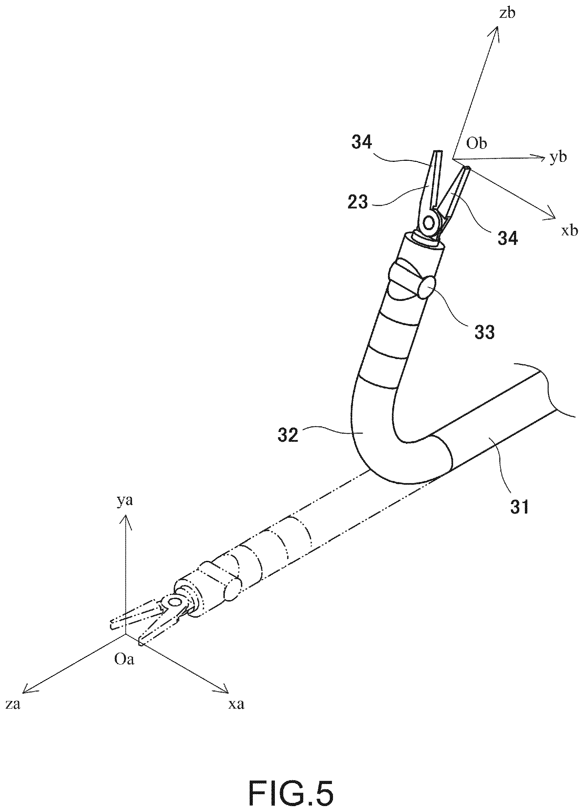

FIG. 5 shows an example of an operation of the bendable joint 32. For example, at a time point at which the operation mode is set to the bendable joint operation mode, when the approach vector of a rotation matrix representing the posture of the surgical instrument 23 in the three-dimensional orthogonal reference coordinate system on the operation unit 1 side is za = (zax, zay, zaz) = (0, 0, 1), the bendable joint operation command generator 56 calculates an initial bending angle as tan (0 / 1) / 2π × 360 = 0°. Then, the surgeon S operates the handle 65. When, as a result of the operation of the handle 65 by the surgeon S, the approach vector in the three-dimensional orthogonal reference coordinate system on the operation unit 1 side changes into zb = (zbx, zby, zbz) = (0.51, -0.32, 0.8), the bendable joint operation command generator 56 calculates the current bending angle as tan (-0.32 / 0.8) / 2π × 360 = -24.2°. Then, the bendable joint operation command generator 56 calculates a difference between the current bending angle and the initial bending angle, i.e., -24.2° -0° = -24.2°. Thereafter, the bendable joint operation command generator 56 generates a command to bend the bendable joint 32 by -24.2°. The controller 3 outputs the generated command to the bendable joint driver 26. In response, the bendable joint driver 26 bends the bendable joint 32 by -24.2°. In this manner, the bendable joint 32 can be bent, and the posture of the entire second arm 22 can be changed.

Consequently, as shown in FIG. 6, a direction in which the surgical instrument 23 accesses the surgical site T can be changed greatly, which makes it possible to allow the surgical instrument 23 to access the portion A of the surgical site T while preventing interference between the instrument manipulator 20 and the other portions of the surgical site T.

As a result of the surgeon S removing the foot from the operating pedal 43, the operation mode setter 54 sets the operation mode to the normal operation mode. This allows the surgeon S to smoothly shift to an operation of moving the surgical instrument 23 again.

As described above, in the surgical operation system 100, since the instrument manipulator 20 includes the bendable joint 32, the motion range of the distal end portion of the instrument manipulator 20 can be widened, and the degree of freedom of posture of the instrument manipulator 20 can be increased. This makes it possible to bring the instrument manipulator 20 into such a posture as to avoid interference with the surrounding environment.

Since the operation mode can be switched from the normal operation mode to the bendable joint operation mode by using the operating pedal 43, a smooth shift to an operation of the bendable joint 32 can be made. Since the bending angle of the bendable joint 32 can be changed by using the handle 65, an operation of the bendable joint 32 can be performed smoothly. In this manner, an increase in the operational burden on the surgeon S due to the addition of the operation of the bendable joint 32 can be suppressed. This makes it possible to reduce the surgery time.

### (Embodiment 2)

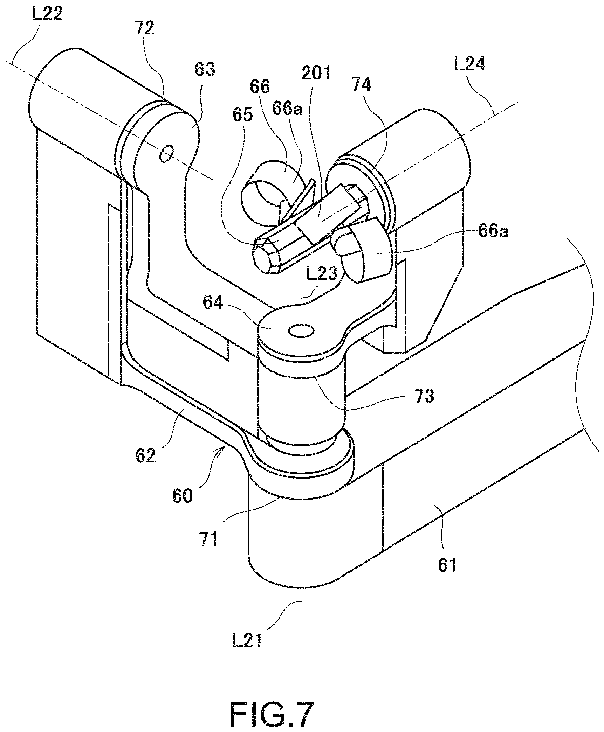

Hereinafter, a configuration and operations according to Embodiment 2 are described focusing on differences from Embodiment 1. FIG. 7 is an essential-part enlarged view showing a configuration example of a distal-side portion of a hand control unit of a surgical operation system according to Embodiment 2.

In the present embodiment, the master console 2 includes, instead of the operating pedal 43, a slide switch 201 provided on the handle 65. The slide switch (a bending angle inputter) 201 is mounted between the pair of annular bodies 66a of the instrument motion inputter 66, and is a switch that can be operated with the index finger of the surgeon S. The slide switch 201 is a switch that receives an operation of inputting a bending angle of the bendable joint 32. The slide switch 201 is slidable in the forward-backward direction. With the slide switch 201, an operation of sliding the slide switch 201 in one sliding direction to decrease the bending angle in accordance with the sliding of the slide switch 201, i.e., an operation of stretching the bendable joint 32 straight, can be inputted. Also, with the slide switch 201, an operation of sliding the slide switch 201 in the other sliding direction to increase the bending angle in accordance with the sliding of the slide switch 201, i.e., an operation of bending the bendable joint 32, can be inputted. The bending angle inputted to the slide switch 201 is outputted to the controller 3.

Then, in the present embodiment, at the time of changing the bending angle of the bendable joint 32, the slide switch 201 is operated with the index finger to input the bending angle of the bendable joint 32. In response, the bendable joint operation command generator 56 generates a command to change the bending angle of the bendable joint 32 to the inputted bending angle. The controller 3 outputs the generated command to the bendable joint driver 26. In response, the bendable joint driver 26 drives the bendable joint 32 to change its bending angle to the inputted bending angle.

As described above, in the present embodiment, while holding the handle 65 with the thumb and the middle finger, the surgeon S can operate the slide switch 201 with the free index finger to change the bending angle of the bendable joint 32. Therefore, the surgeon S can smoothly operate the bendable joint 32 without releasing his/her hand from the handle 65, and can smoothly perform the positioning of the surgical instrument 23 to bring the surgical instrument 23 into a reference position and a reference posture. This makes it possible to reduce the burden on the surgeon S and shorten the surgery time.

From the foregoing description, numerous modifications and other embodiments of the present invention are obvious to a person skilled in the art. Therefore, the foregoing description should be interpreted only as an example and is provided for the purpose of teaching the best mode for carrying out the present invention to a person skilled in the art. The structural and/or functional details may be substantially modified without departing from the spirit of the present invention.

### Reference Signs List

- 1: operation unit
- 2: master console
- 3: controller
- 20: instrument manipulator
- 23: surgical instrument
- 25: wrist joint driver
- 26: bendable joint driver
- 31: shaft
- 32: bendable joint
- 33: wrist joint
- 42: hand control unit
- 54: operation mode setter
- 55: normal operation command generator
- 56: bendable joint operation command generator
- 65: handle
- 75: detector
- 100: surgical operation system

## Claims

1. A surgical operation system comprising:
an instrument manipulator including a shaft, a bendable joint, a wrist joint, and a surgical instrument that are connected and arranged in this order from a proximal end portion toward a distal end portion of the instrument manipulator, the instrument manipulator further including
a wrist joint driver that drives the wrist joint based on a normal operation command including a command that specifies a target position of the wrist joint, and
a bendable joint driver that drives the bendable joint based on a bendable joint operation command that specifies a target bending angle of the bendable joint;
a master console including
a base,
a hand control unit including a handle that is displaceable relative to the base and a detector that detects a displacement of the handle, and
an operation mode selection inputter that receives an operation of inputting a selection command to select one operation mode from operation modes including a normal operation mode and a bendable joint operation mode; and
a controller including
an operation mode setter that sets the operation mode to the one operation mode corresponding to the selection command,
a normal operation command generator that generates the normal operation command, and
a bendable joint operation command generator that generates the bendable joint operation command, wherein
in the normal operation mode, the normal operation command generator calculates a target posture of the surgical instrument based on the displacement of the handle, the displacement being detected by the detector, and generates the normal operation command to operate the wrist joint such that the surgical instrument takes the target posture, and
in the bendable joint operation mode, the bendable joint operation command generator generates the bendable joint operation command to change the bending angle of the bendable joint based on the displacement of the handle, the displacement being detected by the detector.

2. The surgical operation system according to claim 1, wherein
the wrist joint is a joint with two degrees of freedom or three degrees of freedom.

3. The surgical operation system according to claim 1 or 2, wherein
the instrument manipulator further includes:
a first arm on a proximal end portion side of the instrument manipulator; and
a first arm driver that drives the first arm based on the normal operation command, which further includes a command that specifies a motion of the first arm, and
in the normal operation mode, the normal operation command generator generates the normal operation command to operate the first arm and the wrist joint such that the surgical instrument takes the target posture at the target position.

4. The surgical operation system according to claim 3, wherein
the normal operation command generator generates the normal operation command to control a position and a posture of the surgical instrument while keeping a center axis of the shaft passing through a preset remote center.

5. A surgical operation system comprising:
an instrument manipulator including a shaft, a bendable joint, a wrist joint, and a surgical instrument that are connected and arranged in this order from a proximal end portion toward a distal end portion of the instrument manipulator, the instrument manipulator further including
a wrist joint driver that drives the wrist joint based on a normal operation command including a command that specifies a target position of the wrist joint, and
a bendable joint driver that drives the bendable joint based on a bendable joint operation command that specifies a target bending angle of the bendable joint;
a master console including a base and a hand control unit, the hand control unit including
a handle displaceable relative to the base,
a detector that detects a displacement of the handle, and
a bending angle inputter on the handle, the bending angle inputter receiving an operation of inputting a bending angle of the bendable joint; and
a controller including
a normal operation command generator that generates the normal operation command, and
a bendable joint operation command generator that generates the bendable joint operation command, wherein
the normal operation command generator calculates a target posture of the surgical instrument based on the displacement of the handle, the displacement being detected by the detector, and generates the normal operation command to operate the wrist joint such that the surgical instrument takes the target posture, and
the bendable joint operation command generator generates the bendable joint operation command to change the bending angle of the bendable joint based on the bending angle of the bendable joint received by the bending angle inputter.

6. A method of controlling a surgical operation system, the surgical operation system including:
an instrument manipulator including a shaft, a bendable joint, a wrist joint, and a surgical instrument that are connected and arranged in this order from a proximal end portion toward a distal end portion of the instrument manipulator, the instrument manipulator further including a wrist joint driver that drives the wrist joint and a bendable joint driver that drives the bendable joint; and
a master console including
a base,
a hand control unit including a handle that is displaceable relative to the base and a detector that detects a displacement of the handle, and
an operation mode selection inputter that receives an operation of inputting a selection command to select one operation mode from operation modes including a normal operation mode and a bendable joint operation mode,
the method comprising:
setting the operation mode to the one operation mode corresponding to the selection command;
in the normal operation mode,
calculating a target posture of the surgical instrument based on the displacement of the handle, the displacement being detected by the detector,
generating a normal operation command to operate the wrist joint such that the surgical instrument takes the target posture, and
driving the wrist joint based on the normal operation command; and
in the bendable joint operation mode,
generating a bendable joint operation command to change a bending angle of the bendable joint based on the displacement of the handle, the displacement being detected by the detector, and
driving the bendable joint based on the bendable joint operation command.
